# EUROPÄISCHE PATENTSCHRIFT

(11) **EP 0 531 950 B1**
(45) Veröffentlichungstag und Bekanntmachung des Hinweises auf die Patenterteilung: **27.09.1995**
(21) Anmeldenummer: 92115360.7
(22) Anmeldetag: 09.09.1992
(51) Int. Cl.: A61C 1/08, B23B 49/00

(54) **Vorrichtung zur Begrenzung der Eindringtiefe eines zahnärztlichen Werkzeugs**
Penetration depth limiting device for a dental workpiece
Dispositif pour limiter la profondeur de pénétration d'un outil dentaire

(30) Priorität: 12.09.1991 DE 4130271
(43) Veröffentlichungstag der Anmeldung: 17.03.1993
(73) Patentinhaber: IMPLA GmbH, Dental-Implantate, D-61191 Rosbach (DE)
(72) Erfinder: Kranjc, Josef, Dipl.-Ing., D-6350 Bad Nauheim-Steinfurth (DE)
(74) Vertreter: Schlagwein, Udo, Dipl.-Ing.

(56) Entgegenhaltungen:
- DE-C- 179 553
- US-A- 2 779 218
- US-A- 3 838 517
- US-A- 4 381 917
- US-A- 4 752 223

## Beschreibung

Die Erfindung betrifft eine Vorrichtung zur Begrenzung der Eindringtiefe eines in einem Bohrerhandstück eingespannten, rotierenden zahnärztlichen Werkzeugs in einen Zahn, eine Zahnwurzel oder den Kieferknochen eines Patienten, gemäß dem ersten Teil von Anspruch 1. Eine derartige Vorrichtung ist aus der US-A-3 838 517 bekannt.

Im menschlichen Kiefer verläuft unterhalb der Zähne jeweils ein Nervenstrang, welcher bei der zahnärztlichen Behandlung auf keinen Fall durchtrennt werden darf. Diese Gefahr ist insbesondere dann gegeben, wenn der Zahnarzt zum Einsetzen eines Implantates in den Kieferknochen ein relativ tiefes Loch bohren oder fräsen muß. Da der Verlauf des Nervenstranges durch eine Röntgenaufnahme feststellbar ist, kann man eine Durchtrennung des Nervenstranges dadurch ausschließen, daß man die mögliche Eindringtiefe des Bohrers oder Fräsers durch einen Anschlag begrenzt.

Bei nicht im dentalen Bereich eingesetzten Bohrmaschinen sind bereits einstellbare Anschläge zur Begrenzung der maximal möglichen Bohrtiefe bekannt. Sie weisen einen höhenverstellbar am Bohrkopf befestigten Stab auf, der sich bei Erreichen der gewünschten Bohrtiefe auf den zu bohrenden Gegenstand aufsetzt.

Wollte man einen solchen Anschlag bei einem zahnärztlichen Bohrer vorsehen, dann müßte hierzu der gesamte Bohrkopf umgestaltet werden. Das erforderte es, daß sich die Zahnärzte neue Bohrerhandstücke zulegen und die bisherigen Bohrerhandstücke nicht mehr verwenden, wenn tief in den Kieferknochen reichende Löcher gebohrt werden sollen.

Der Erfindung liegt das Problem zugrunde, eine möglichst einfache Vorrichtung zum Begrenzen der Eindringtiefe eines zahnärztlichen, rotierenden Werkzeugs zu entwikkeln, die bei üblichen Bohrerhandstücken ohne jede Veränderung der Bohrerhandstücke benutzt werden kann.

Dieses Problem wird erfindungsgemäß durch die in Anspruch 1 definierte Vorrichtung gelöst.

Eine solche Hülse kann einfach auf einen eingespannten Bohrer oder Fräser aufgeschoben und dann am Bohrerhandstück befestigt werden. Danach ist es nur noch möglich, so tief zu bohren oder mit dem Fräser in ein Loch einzudringen, bis sich die freie Stirnfläche der Hülse auf den Rand des Loches, also den Kieferknochen oder einen Zahnbereich, aufsetzt. Die erfindungsgemäße Vorrichtung ist sehr einfach ausgebildet. Sie ist deshalb kostengünstig herstellbar und kann leicht desinfiziert werden.

Die Befestigung der Hülse am Bohrerhandstück ist ohne Werkzeug mit einem einzigen Handgriff möglich, wenn gemäß einer vorteilhaften Ausgestaltung der Erfindung die Halterung durch einen längenelastischen, an zwei diametral gegenüberliegenden Seiten der Hülse befestigten Bügel gebildet ist, dessen Länge so bemessen ist, daß der Bügel mit Vorspannung über den Bohrkopf hinweg auf das Bohrerhandstück geschoben werden kann.

Besonders einfach ist der Bügel gestaltet, wenn er durch eine schraubenförmig gewickelte Zugfeder gebildet ist.

Die Vorrichtung ist insgesamt besonders einfach gestaltet, wenn der Bügel in Bohrungen eines am oberen, dem Bohrkopf zugewandten Ende der Hülse vorgesehenen Flansches eingehangen ist.

Der Flansch behindert nicht die Wasserzuführung über eine Leitung und Spritzdüse zur Spitze des Bohrers oder Fräsers, wenn der Flansch auf einer Seite des Bügels im Durchmesser verringert ist.

Man könnte für unterschiedliche Eindringtiefen unterschiedlich lange Hülsen vorsehen, so daß der Zahnarzt nach Festlegung der richtigen Eindringtiefe sich die richtige Hülse heraussuchen und dann auf den Bohrer oder Fräser schieben müßte. Es ist jedoch möglich, mit nur zwei verschieden bemessenen Vorrichtungen auszukommen, wenn gemäß einer besonders vorteilhaften Ausgestaltung der Erfindung die Hülse aus einem mit Außengewinde versehenen Innenteil und einem darauf mit Innengewinde geschraubten Außenteil besteht. Eine solche Hülse kann durch mehr oder weniger weites Aufschrauben des Außenteiles auf das Innenteil in ihrer Länge verändert und dem gewünschten Maß exakt angepaßt werden.

Die Erfindung läßt zahlreiche Ausführungsformen zu. Eine davon ist in der Zeichnung dargestellt und wird nachfolgend beschrieben. Diese zeigt in
- Fig.1: eine perspektivische Ansicht eines Bohrerhandstückes mit der erfindungsgemäßen Vorrichtung,
- Fig.2: einen senkrechten Schnitt durch die Vorrichtung,
- Fig.3: eine Draufsicht auf die Vorrichtung.

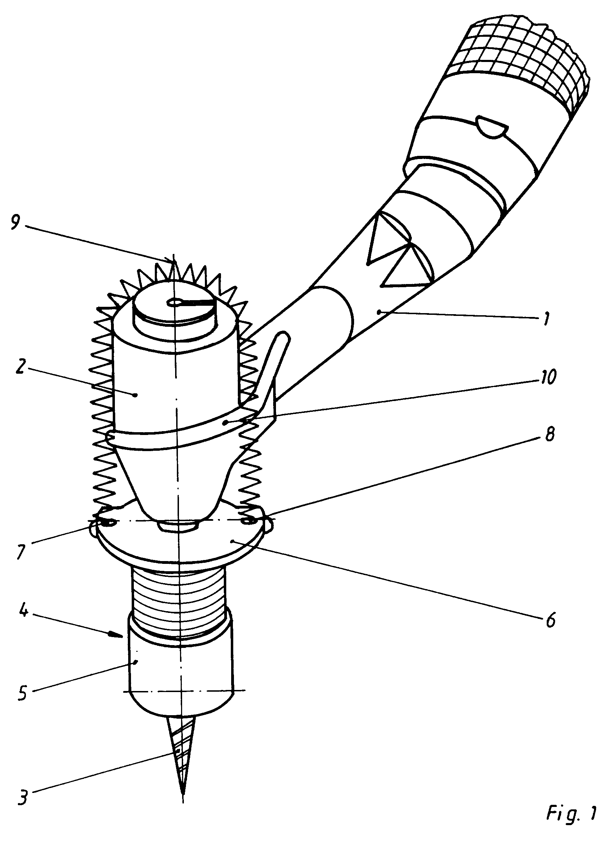

In der Figur 1 ist ein Bohrerhandstück 1 mit einem Bohrkopf 2 und einem darin eingespannten rotierenden Werkzeug 3 gezeigt. Bei diesem Werkzeug 3 kann es sich zum Beispiel um einen Bohrer oder einen Fräser handeln.

Wichtig für die Erfindung ist eine Vorrichtung 4, mit der die maximal mögliche Bohr- oder Frästiefe begrenzt wird. Diese Vorrichtung 4 hat eine Hülse 5, welche am oberen Ende einen Flansch 6 mit zwei diametral gegenüberliegenden Bohrungen 7, 8, in welchen ein durch eine schraubenlinienförmig gewickelte Zugfeder ausgebildeter Bügel 9 eingehangen ist. Teilweise dargestellt ist eine entlang des Bohrerhandstückes 1 verlaufende Leitung 10, über welche zur Spitze des rotierenden Werkzeugs 3 spritzendes Wasser zugeführt wird.

Die Hülse 5 ist von unten her auf das rotierende Werkzeug 3 so weit aufgeschoben, daß der Flansch 6 gegen den Bohrkopf 2 anliegt. Sie wird in dieser Position durch den Bügel 9 gehalten, welcher über den Bohrkopf 2 gelegt ist und dadurch die Hülse 5 nach oben hin vorspannt.

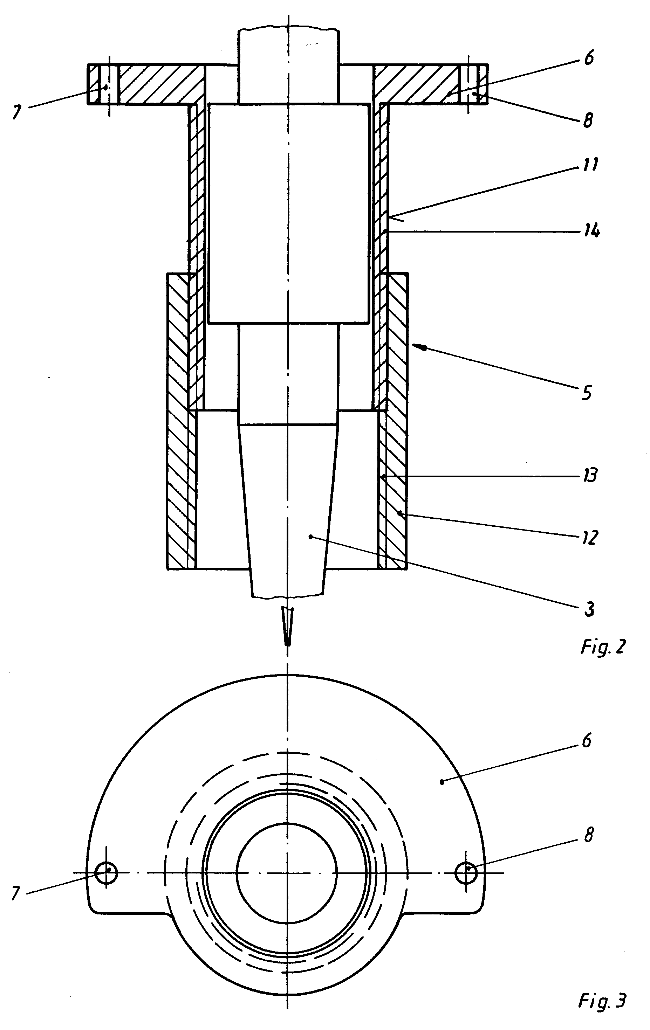

In Figur 2 ist gezeigt, daß die Hülse 5 aus einem Innenteil 11 mit Außengewinde 14 und einem darauf mit Innengewinde 13 aufgeschraubten Außenteil 12 gebildet ist. Der Flansch 6 mit seinen Bohrungen 7, 8 ist einstückig mit dem Innenteil 11 ausgebildet. Mittig in der Hülse 5 ist in Figur 2 das rotierende Werkzeug 3 dargestellt.

Die Figur 3 zeigt, daß der Flansch 6 im wesentlichen nur auf einer Seite der Bohrungen 7, 8 vorgesehen ist. Dadurch kann das über die in Figur 1 gezeigte Leitung 10 zugeführte Wasser vom Flansch 6 unbehindert zum rotierenden Werkzeug 3 gelangen.

### Auflistung der verwendeten Bezugszeichen

- 1: Bohrerhandstück
- 2: Bohrkopf
- 3: Werkzeug
- 4: Vorrichtung
- 5: Hülse
- 6: Flansch
- 7: Bohrung
- 8: Bohrung
- 9: Bügel
- 10: Leitung
- 11: Innenteil
- 12: Außenteil
- 13: Innengewinde
- 14: Außengewinde

## Patentansprüche

1. Vorrichtung zur Begrenzung der Eindringtiefe eines in einem Bohrerhandstück (1) eingespannten, rotierenden zahnärztlichen Werkzeugs (3) in einen Zahn, eine Zahnwurzel oder den Kieferknochen eines Patienten wobei die Vorrichtung eine Halterung (9) zum Verbinden mit dem Bohrerhandstück (1) aufweist, **dadurch gekennzeichnet**, daß sie als auf das rotierende Werkzeug (3) aufschiebbare und gegen den feststehenden Teil eines Bohrerhandstückes (1) abstützbare Hülse (5) ausgebildet ist.

2. Vorrichtung nach Anspruch 1, **dadurch gekennzeichnet,** daß die Halterung durch einen längenelastischen, an zwei diametral gegenüberliegenden Seiten der Hülse (5) befestigten Bügel (9) gebildet ist, dessen Länge so bemessen ist, daß der Bügel (9) mit Vorspannung über den Bohrkopf (2) hinweg auf das Bohrerhandstück (1) geschoben werden kann.

3. Vorrichtung nach Anspruch 2, **dadurch gekennzeichnet**, daß der Bügel (9) durch eine schraubenförmig gewickelte Zugfeder gebildet ist.

4. Vorrichtung nach zumindest einem der vorangehenden Ansprüche, **dadurch gekennzeichnet**, daß der Bügel (9) in Bohrungen (7, 8) eines am oberen, dem Bohrkopf (2) zugewandten Ende der Hülse (5) vorgesehenen Flansches (6) eingehangen ist.

5. Vorrichtung nach Anspruch 4, **dadurch gekennzeichnet**, daß der Flansch (6) auf einer Seite des Bügels (9) im Durchmesser verringert ist.

6. Vorrichtung nach zumindest einem der vorangehenden Ansprüche, **dadurch gekennzeichnet**, daß die Hülse (5) aus einem mit Außengewinde (14) versehenen Innenteil (11) und einem darauf mit Innengewinde (13) geschraubten Außenteil (12) besteht.

## Claims

1. Device for limiting the depth of penetration of a rotating dental instrument (3), fixed in a drill handle (1), into a tooth, a tooth root or a patient's jawbone, wherein the device has a mounting (9) for connection to the drill handle (1), characterised in that it is formed as a tube (5) which can be pushed onto the rotating tool (3) and can be supported against the stationary part of a drill handle (1).

2. Device according to Claim 1, characterised in that the mounting is formed by a longitudinally resilient hoop (9) which is fastened on two diametrally opposed sides of the tube (5) and of which the length is calculated such that the hoop (9) can be pushed over the drill head (2) onto the drill handle (1) with initial tension.

3. Device according to Claim 2, characterised in that the hoop (9) is formed by a helically wound tension spring.

4. Device according to at least one of the preceding claims, characterised in that the hoop (9) is installed in orifices (7, 8) in a flange (6) provided at the upper end of the tube (5) facing the drill head (2).

5. Device according to Claim 4, characterised in that the flange (6) is reduced in diameter on one side of the hoop (9).

6. Device according to at least one of the preceding claims, characterised in that the tube (5) consists of an internal part (11) provided with an external thread (14) and an external part (12) screwed thereon by an internal thread (13).

## Revendications

1. Dispositif pour délimiter la pénétration dans une dent, une racine dentaire ou l'os maxillaire d'un patient, d'un outil (3) de chirurgie dentaire, rotatif, monté dans la pièce à main (1) d'un foret, le dispositif présentant un support (9) destiné à l'assemblage avec ladite pièce à main (1), caractérisé en ce que ledit dispositif est réalisé sous la forme d'une douille (5) s'appuyant contre la partie fixe de la pièce à main (1) d'un foret, et pouvant être emmanchée sur l'outil rotatif (3).

2. Dispositif selon la revendication 1, caractérisé en ce que le support est constitué par un étrier (9), élastique en longueur, fixé sur deux côtés diamétralement opposés de la douille (5), la longueur dudit étrier étant mesurée de telle sorte qu'il peut être poussé, sous tension initiale, sur ladite pièce à main (1), en passant par dessus la tête du foret (2).

3. Dispositif selon la revendication 2, caractérisé en ce que l'étrier est constitué d'un ressort de traction enroulé en forme d'hélice.

4. Dispositif selon au moins l'une des revendications précédentes, caractérisé en ce que l'étrier (9) est accroché dans des trous (7, 8) d'une bride (6) prévue à l'extrémité supérieure de la douille (5) orientée vers la tête de foret (2).

5. Dispositif selon la revendication 4, caractérisé en ce que la bride (6) présente un diamètre réduit sur un côté de l'étrier (9).

6. Dispositif selon au moins l'une des revendications précédentes, caractérisé en ce que la douille (5) est constituée d'une partie intérieure (11) munie d'un filetage extérieur (14) et d'une partie extérieure (12) vissée sur celui-ci à l'aide d'un filetage intérieur (13).
